# EUROPÄISCHE PATENTSCHRIFT

(11) **EP 2 898 056 B1**
(45) Veröffentlichungstag und Bekanntmachung des Hinweises auf die Patenterteilung: **02.08.2017**
(21) Anmeldenummer: 13762470.6
(22) Anmeldetag: 12.09.2013
(51) Int. Cl.: C12M 1/06, B01F 3/04, C12M 1/08, C12M 1/02, B01F 15/06, B01F 7/22, B01F 7/16

(54) **BIOREAKTOR UND BAUGRUPPE ZUM EINBAU IN EINEN BIOREAKTOR**
BIOREACTOR AND ASSEMBLY FOR INSTALLATION IN A BIOREACTOR
BIORÉACTEUR ET MODULE À INCORPORER DANS UN BIORÉACTEUR

(30) Priorität: 18.09.2012 AT 503972012
(43) Veröffentlichungstag der Anmeldung: 29.07.2015
(73) Patentinhaber: Biogas Systems GmbH, 7111 Parndorf (AT)
(72) Erfinder: DAUSER, Hermann, A-5071 Wals Siezenheim (AT)
(74) Vertreter: Babeluk, Michael
(86) Internationale Anmeldenummer: PCT/EP2013/068941
(87) Internationale Veröffentlichungsnummer: WO 2014/044598

(56) Entgegenhaltungen:
- WO-A1-01/39872
- DE-A1- 4 130 492
- GB-A- 2 288 796
- US-A- 4 036 699
- US-A- 4 927 530

## Beschreibung

Die Erfindung betrifft einen Bioreaktor, insbesondere Fermentationsreaktor, mit einem zylindrischen Reaktortank mit vertikaler Achse und einem in einem Strömungsleitrohr angeordneten, im Wesentlichen vertikal nach unten fördernden Rührwerk, sowie einem im Inneren des Reaktortanks angeordneten Wärmetauscher, wobei der Wärmetauscher im Bereich der Austrittsöffnung des Strömungsleitrohrs angeordnet ist und in Bezug auf die vertikale Achse des Reaktortanks radial nach außen weisende, vertikale Strömungsleitelemente aufweist. Weiters betrifft die Erfindung eine Baugruppe zum Einbau in einen Bioreaktor, insbesondere Fermentationsreaktor, mit einem zylindrischen Reaktortank, wobei die Baugruppe ein Strömungsleitrohr mit integriertem, vertikal nach unten förderndem Rührwerk aufweist.

Fermentationsreaktoren, beispielsweise für Biogasanlagen, haben typischerweise Volumina zwischen 500 m³ und 5000 m³ und werden großteils in zwei Bauarten ausgeführt:
a) Flache zylindrische Reaktoren, mit Wassertiefen zwischen 5 m und 8 m, und Durchmessern bis zu 30 m, umgewälzt durch Schrägwellen-Rührwerke (mit Bodenlagern) oder Tauchrührwerke mit horizontaler Strömung. Die Ausführung erfolgt teilweise auch als Außenring um einen innen liegenden Reaktor mit kreisförmigem Grundriss (sog. Ring-Anlage). Als Heizelemente werden in der Regel innen liegende Heizregister aus dünnen Rohren oder Wandheizungen eingesetzt, teilweise auch externe Wärmetauscher-Kreisläufe.
b) Hohe zylindrische Reaktoren, mit Höhen und Durchmessern bis zu 20 m, umgewälzt durch vertikale Mischer mit einem oder mehreren horizontalen Rührebenen, und nach Erfordernis einem Bodenlager für die Rührwelle. Beheizt werden diese ebenfalls mit Wandheizungen oder externen Wärmetauscher-Kreisläufen.

Fermentationsreaktoren nach a) zeichnen sich durch einen hohen erforderlichen Mischenergie-Eintrag für eine gleichmäßige Durchströmung aus, sowie (teilweise) undefinierte Strömungszustände und große Totzonen, insbesondere mit der Neigung zur Bildung von Schwimmteppichen aus unfermentierter Biomasse, die den Anlagenbetrieb nachhaltig stören. Innen liegende Heizregister sind meist sehr filigran, und stets in Gefahr durch Anhaftungen und Strömungskräfte abgerissen zu werden. In der Wand verlegte Heizungsleitungen sind im Leckagefall sehr schwierig zu reparieren, und externe Wärmetauscherkreisläufe sind vergleichsweise teuer in der Investition und aufgrund permanenter Umwälzung ebenfalls teuer im Betrieb.

Aus der CN 01334477 Y ist ein Fermentationsreaktor bekannt geworden, der einen vertikal stehenden, zylindrischen Reaktortank aufweist, wobei im Inneren ein zentrales Strömungsleitrohr angeordnet ist, das als Wärmetauscher ausgeführt ist. Im Inneren des Strömungsleitrohres befindet sich ein nach unten förderndes Rührwerk, dessen Antriebsachse nach oben aus dem Reaktortank herausgeführt ist und eine außenliegende Antriebsquelle aufweist. Diese Bauart ist jedoch hauptsächlich für kleinere Reaktortanks mit einem Fassungsvermögen bis 100 m³ oder 150 m³ vorstellbar.

Die WO 01/39872 A1 zeigt ebenfalls einen Reaktorbehälter mit einem zentralen Strömungsleitrohr, in dem sich ein nach unten förderndes Rührwerk befindet, dessen Antriebsachse nach oben aus dem Reaktortank herausgeführt ist. Der Reaktorbehälter weist einen Heizmantel auf, der in die konische Bodenwandung und den unteren Teil der Seitenwand integriert ist. Der Wärmetauscher ist somit Bestandteil der Bodenwand und der Seitenwand des Reaktorbehälters. Weiters sind radial nach außen weisende, vertikale Strömungsleitelemente vorgesehen, die als Strömungsbrecher für die zirkularen Strömungsanteile dienen.

Aus der GB 2 288 796 A ist eine Baugruppe zum Einbau in einen zylindrischen Reaktortank bekannt, wobei die Baugruppe ein im eingebauten Zustand vertikales Strömungsleitrohr mit einem vertikal nach unten fördernden Rührwerk aufweist.

Fermentationsreaktoren nach b) haben aufgrund der definierten Bedingungen und geometrischen Anordnung mit zentralem Vertikalrührwerk verbesserte Strömungseigenschaften, in der Praxis jedoch neigen auch diese Systeme zu erheblichen Betriebsproblemen:
Aufgrund der oft großen Behälterdimensionen und der hohen Viskosität des Mediums kann meist keine durchgehende Strömungsschlaufe über die gesamte Reaktorhöhe ausgebildet werden, sondern es entstehen bei mehreren Rührebenen in sich geschlossene Zirkulationswirbel, oder typisch bei nur einer Rührebene strömungsfreie Zonen am Reaktorboden und besonders an der Wasseroberfläche.

Die sich ergebenden Schwimmteppiche führen meist zu erheblichen Betriebsstörungen. Für die Heizungssysteme gilt im Allgemeinen dasselbe wie unter a) beschrieben.

Als typische Konstruktionsmaterialien werden z.B. im Bereich der Biogasfermenter folgende Werkstoffe eingesetzt:
- Stahlbeton, teilweise korrosionsfest beschichtet
- Edelstahl, teilweise in extrem dünnwandiger Wickelfalz-Konstruktion
- Stahl emailliert, in Plattenbauweise mit Schraubverbindung

Hohe Fermenter gemäß b) werden meist aus Edelstahl oder emailliertem Stahl auf einer Beton-Fundamentplatte hergestellt, da die Bauweise aus Beton zu hohe Kosten durch enorme Wandstärken bedingt.

Die Schwachstelle insbesondere der dünnwandigen Wickelfalz-Bauweise sowie der Emailbeschichteten Plattenbauweise ist die Gefahr von Undichtigkeiten oder Beschädigungen des Wandmaterials durch die Befestigung interner Bauteile (Heizung, Strömungsleitelemente), sowie teilweise eine unzureichende statische und konstruktive Festigkeit für die Anbringung zusätzlicher Ausrüstungsteile.

Aufgabe der Erfindung ist es, einen Bioreaktor, insbesondere Fermentationsreaktor, mit einem zylindrischen Reaktortank auf konstruktiv einfache Weise derart auszurüsten, dass die Umwälzung des Reaktorinhalts und die Temperierung des Reaktors optimiert, und ein flexibler Einsatz für unterschiedliche Fermenter-Materialien und Konstruktionen ermöglicht wird.

Diese Aufgabe wird erfindungsgemäß dadurch gelöst, dass mehrere, vorzugsweise alle Strömungsleitelemente jeweils ein von einer ersten Ringleitung gespeistes, oberes Rohr und ein von einer zweiten Ringleitung gespeistes, unteres Rohr aufweisen, wobei die beiden Rohre jedes Strömungsleitelementes zur Zu- und Abfuhr eines Heiz- oder Kühlmediums dienen und zwischen den beiden Rohren ein vertikales Leitblech angeordnet ist.

Bevorzugt ist zwischen der Austrittsöffnung des Strömungsleitrohrs und dem zentralen Bereich des Wärmetauschers ein Strömungsleitkegel zur Umlenkung der vom Rührwerk erzeugten Vertikalströmung in eine nach außen gerichtete Radialströmung angeordnet.

Zur Nachrüstung bestehender Bioreaktoren eignet sich erfindungsgemäß bevorzugt eine Baugruppe, die ein Strömungsleitrohr mit integriertem, vertikal nach unten förderndem Rührwerk, ausgangsseitig des Strömungsleitrohrs, einen Strömungsleitkegel zur Umlenkung der vom Rührwerk erzeugten Vertikalströmung in eine nach außen gerichtete Radialströmung und einen Wärmetauscher mit radial nach außen weisenden Strömungsleitelementen aufweist.

Das offene vertikale Strömungsleitrohr beginnt unterhalb des Wasserspiegels, und endet beispielsweise bei einem Drittel der Reaktorhöhe. Das Strömungsleitrohr ist durch mehrere Standfüße auf der (ebenen) Fundamentplatte befestigt.

Im Strömungsleitrohr ist ein (langsam laufendes) spezielles Rührwerk angeordnet, das im Wesentlichen keine radial abgehende Strömung bedingt, sondern gezielt eine kanalisierte Strömung vertikal nach unten erzeugt.

Für die Installation des Rührwerkes sind grundsätzlich zwei Alternativen möglich:
1) Das Rührwerk ist mit vertikaler Achse und externem Antriebsmotor (außerhalb des Reaktors) ausgeführt, wobei das Mischelement (Propeller) vorzugsweise im oberen Bereich des Strömungsleitrohres liegt, wodurch nur eine sehr kurze Rührwelle erforderlich ist. Diese Bauweise kann z.B. bei emaillierten Stahl- oder stabilen Edelstahl-Behältern eingesetzt werden, die über einen stabilen, tragfähigen Kronenring verfügen.
2) Das Rührwerk wird als Tauchrührwerk ausgeführt, und mit gleicher Strömungsrichtung, jedoch vorzugsweise am unteren Ende des Strömungsleitrohres installiert. Die Befestigung des Rührwerkes erfolgt in diesem Fall in einer (speichenförmigen) Haltekonstruktion, die mit den Standfüßen des Strömungsleitrohres verbunden sein kann.

In beiden Fällen überträgt das Rührorgan keinerlei Kräfte auf das Strömungsleitrohr selbst, und erzeugt eine kontinuierliche vertikal gerichtete Abwärtsströmung im Strömungsleitrohr.

Variante 2) ermöglicht auch die Ausrüstung von Leichtbaubehältern, die über keine tragfähige Dachkonstruktion verfügen.

Die Erfindung wird im Folgenden anhand von Zeichnungen einer beispielhaften Ausführungsvariante näher erläutert. Es zeigen:
- Fig. 1: einen erfindungsgemäßen Bioreaktor in einem Axialschnitt;
- Fig. 2: den Bioreaktor gemäß Fig. 1 in einer Schnittdarstellung gemäß Linie II-II in Fig. 1; sowie
- Fig. 3: ein Detail des Bioreaktors gemäß Fig. 1 in einer dreidimensionalen Darstellung.

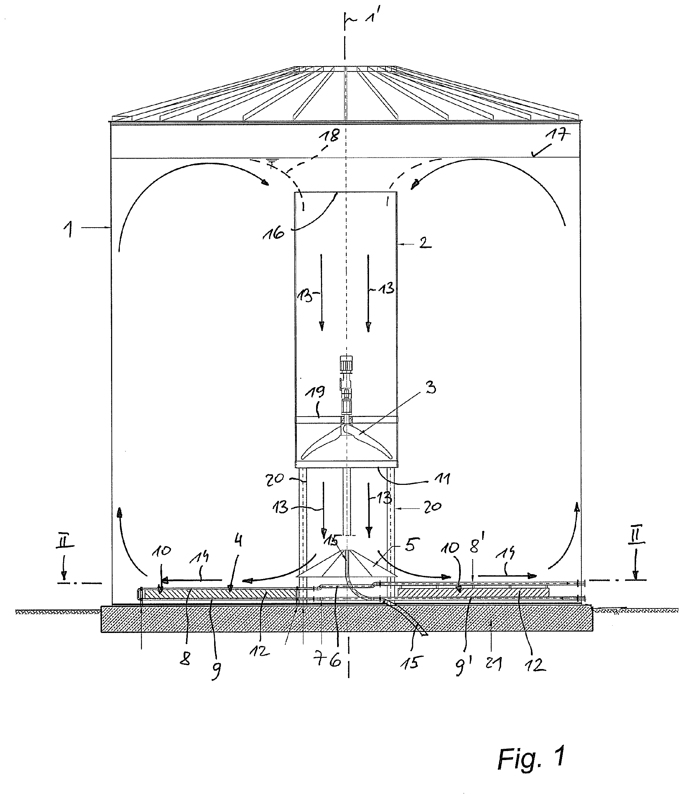

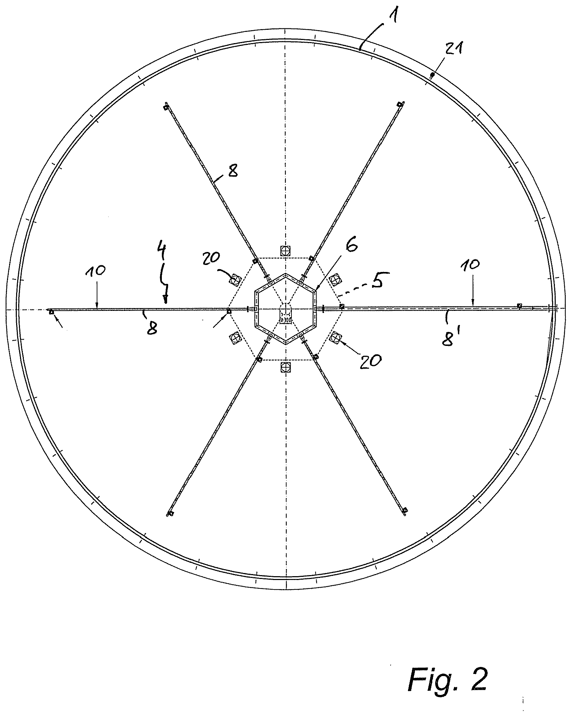

Der in den Fig. 1 bis Fig. 3 dargestellte Bioreaktor weist einen zylindrischen Reaktortank 1 mit vertikaler Achse 1' auf, mit einem in einem zentralen Strömungsleitrohr 2 angeordneten, im Wesentlichen vertikal nach unten fördernden Rührwerk 3, wobei im Bereich der Austrittsöffnung 11 des Strömungsleitrohrs 2 unter Zwischenschaltung eines Strömungsleitkegels 5 ein Wärmetauscher 4 angeordnet ist. Der Wärmetauscher 4 weist in Bezug auf die vertikale Achse 1' des Reaktortanks 1 radial nach außen weisende Strömungsleitelemente 10 auf.

Die einzelnen Strömungsleitelemente 10 weisen jeweils ein von einer ersten, zentralen Ringleitung 6 gespeistes, oberes Rohr 8 und ein von einer zweiten zentralen Ringleitung 7 gespeistes, unteres Rohr 9 auf, wobei die beiden Rohre 6, 8 jedes Strömungsleitelementes 10 zumindest am wandseitigen Ende eine Strömungsverbindung aufweisen und zur Zu- und Abfuhr eines Heiz- oder Kühlmediums dienen. Zwischen den beiden Rohren 6, 8 ist ein vertikal ausgerichtetes Leitblech 12 angeordnet, mit welchem zirkulare Strömungsanteile unterbunden werden können. Der Strömungsleitkegel 5 dient zur Umlenkung der vom Rührwerk 3 erzeugten Vertikalströmung 13 in eine nach außen gerichtete Radialströmung 14. Die beiden zentralen Ringleitungen 6 und 7 können auch in einen Strömungsleitkegel 5 (bzw. Strömungsleitkörper), z.B. aus einem Kunststoffmaterial, integriert oder eingeschäumt sein.

Bevorzugt ist die vertikale Lage der zentralen Eintrittsöffnung 16 des Strömungsleitrohres 2 auf die Höhe des Füllstandes des Substrates im Reaktortank 1 derart abgestimmt, dass sich beim Betrieb des Rührwerks 3 an der Substratoberfläche 17 eine Strömungstrombe 18 ausbildet.

Der Zufluss des Substrates zum Strömungsleitrohr 2 erfolgt aus der oberen Flüssigkeitszone, also direkt an der Substratoberfläche 17. Die Strömungsintensität an der Überfallkante des Strömungsleitrohres 2 kann durch einen einstellbaren Wasserspiegel optimiert werden, wodurch höhere und niedrigere Zuströmgeschwindigkeiten bzw. auch wechselnde Zustände erzeugt werden können.

Dies zusammen mit der Ausbildung einer Strömungstrombe 18 am oberen Ende des Strömungsleitrohres 2 dient der effektiven Untermischung von sich möglicherweise an der Substratoberfläche 17 bildenden Schwimmteppichen aus organischem Material.

Am unteren, austrittseitigen Ende des Strömungsleitrohres 2 sorgt der zentral angeordnete, (rein kegelförmige oder hyperboloide) Strömungsleitkegel 5 für eine gleichmäßige horizontale, Radialströmung 14 zum Außenbereich des Reaktors hin.

Durch die strömungsgünstige Gestaltung des Strömungsleitkegels 5 werden Geschwindigkeitsverluste durch den Austritt und die Umlenkung der Strömung minimiert.

Für die Zufuhr von Rohsubstraten in den Reaktortank 1 ist eine im Bereich der Spitze des Strömungsleitkegels 5 einmündende Substratzufuhrleitung 15 vorgesehen, da an dieser Stelle die hohe Mischenergie für eine optimale Einmischung des Substrates sorgt.

Die horizontale Umlenkung der Strömung am Strömungsleitkegel 5 führt das Medium mit hoher Fließgeschwindigkeit entlang von den im Bodenbereich des Reaktortanks 1 radial angeordneten Strömungsleitelementen 10, die verhindern, dass das Medium, initiiert durch die Rotationskomponente der Rührwerksbewegung, in eine für den Reaktorbehälter destabilisierende Rotationsbewegung gerät.

Durch die hohe Anströmgeschwindigkeit direkt nach dem Austritt aus dem Strömungsleitrohr 2 ergibt sich ein wesentlich verbessertes Wärmeübergangsverhalten zwischen Wärmetauscher 4 und Medium als bei üblichen, nur schwach angeströmten an der Außenwand angebrachten Wandheizungen.

Die Heizregister ausgebildeten Strömungsleitelemente 10 werden über die zentral unter dem Strömungsleitkegel 5 angeordnete Ringleitung 6 (Verteilerkranz) versorgt, auch der Abfluss aus den Heizregistern erfolgt über eine zentrale Ringleitung 7 (Sammelkranz). Diese Anordnung reduziert die erforderlichen Verteilerleitungen auf ein Minimum, und vermeidet die Anordnung strömungsstörender Ringleitungen im Wandbereich. Zur Anbindung der beiden Ringleitungen 6, 7 an die externen Versorgungseinrichtungen kann die Zu- und Abfuhr des Heiz- oder Kühlmediums über das obere 8' und das untere Rohr 9' eines der Strömungsleitelemente 10 erfolgt, dessen beiden Rohre 8', 9' durch die Wand des Reaktortanks 1 hindurchgeführt sind. Die Anbindung kann auch von unten durch die Fundamentplatte 21 erfolgen.

Im Außenbereich des Reaktortanks 1 erfolgt bei abgeschwächter Strömungsgeschwindigkeit eine Umlenkung der Radialströmung 14 nach oben, und das Medium steigt auf zur Beckenoberfläche, um dort wieder in das zentrale Strömungsleitrohr 2 eingezogen zu werden.

Die während des Fermentationsprozesses erzeugten gasförmigen Produkte, z.B. methanhaltiges Biogas, unterstützen diese kontinuierliche Strömung durch ihr Aufsteigen zur Substratoberfläche 17. Da keine Zwischenwirbel mit unterschiedlichen Strömungsrichtungen vorhanden sind, kommt es zu keiner Anreicherung von Mikroblasen im System oder zur Bildung von Gasblasen-Kissen, wie dies bei Vertikalrührwerken (mit mehreren Rührebenen) und horizontalem Energieeintrag bekannt ist.

Der Gasaustritt erfolgt an der bewegten Wasseroberfläche, spätestens jedoch in der turbulenten Strömungszone am Eintritt in das zentrale Strömungsleitrohr 2.

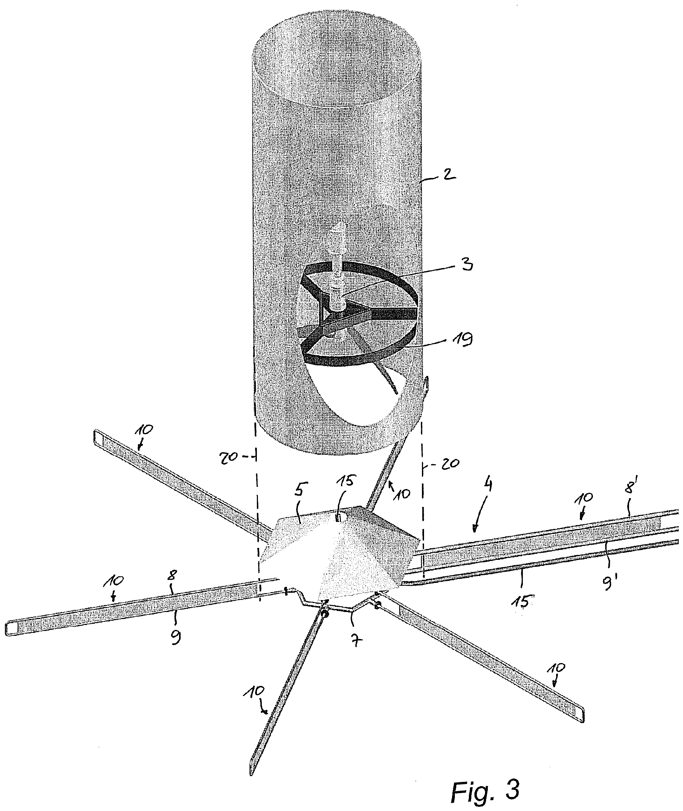

Die in Fig. 3 dargestellte Baugruppe zum Einbau in einen Fermentationsreaktor weist folgende Bestandteile auf:
- ein vertikales, zentrales Strömungsleitrohr 2 mit integriertem, vertikal nach unten förderndem Rührwerk 3,
- ausgangsseitig des Strömungsleitrohrs 2 einen Strömungsleitkegel 5 zur Umlenkung der vom Rührwerk 3 erzeugten Vertikalströmung in eine nach außen gerichtete Radialströmung und
- einen Wärmetauscher 4 mit radial nach außen weisenden Strömungsleitelementen 10, die als Heiz- oder Kühlregister ausgebildet sind.

Durch die Befestigung der Strömungsleitelemente 10 direkt am Boden des Reaktortanks 1 oder auf der Fundamentplatte 21 entfällt jegliche Verbindung zur Behälterwand, so dass keine kritischen Befestigungspunkte mehr bestehen.

Über die Rohre 8, 9 der Strömungsleitelemente 10 kann ein Heiz- oder Kühlmedium zugeführt (Vor- und Rücklauf) werden, die vertikalen Leitbleche 12 können als durchströmte Hohlkörper ausgeführt sein.

Die erfindungsgemäße Vorrichtung erzeugt durch die Kombination eines zentralen Strömungsleitrohres 2 mit einem vertikal nach unten fördernden Rührwerk 3 und einem radial nach außen ableitenden Wärmetauscher 4 eine optimale, gleichmäßige Durchmischung für vertikale Fermentationsreaktoren.

Die Anordnung der Rührorgane optimiert den zur Umwälzung erforderlichen Energieeintrag, und ermöglicht eine flexible Ausrüstung verschiedener Reaktorkonstruktionen, auch wenn diese keine zusätzlichen Lasten aufnehmen können.

Insbesondere gilt dies für die beschriebene Ausrüstung des Systems mit einem Tauchmotor- Rührwerk.

Das Strömungsrohr 2 bildet eine funktionale Einheit mit dem Strömungsleitkegel 5, und daran anschließenden radial am Beckenboden angeordneten Strömungsleitelementen 10, die gleichzeitig als durchströmte Heiz- oder Kühlregister verwendet werden. Diese Kombination erfüllt den Zweck der Vermeidung von Rotationsströmungen und erzielt gleichzeitig aufgrund der hohen Anströmgeschwindigkeit einen effektiven Wärmeübergang auf das Medium.

Das Rührwerk 3 ist gemäß Fig. 3 als Tauchrührwerk ausgeführt, welches von einem Speichenkranz 19 im Strömungsrohr 2 zentriert ist und sich vorzugsweise an Standfüßen 20 des Strömungsleitrohres 2 abstützt. An den Standfüßen 20 kann auch der Strömungsleitkegel 5 befestigt sein.

Die modulare Baugruppe kann beispielsweise direkt auf der Fundamentplatte 21 des Fermentationsreaktors aufgebaut werden, wenn erforderlich ohne Kontakt mit oder Befestigung an der Wand- oder Dachkonstruktion des Reaktortanks. Dies ermöglicht sowohl optimierte Neuanlagen als auch einfache Nachrüstungen bestehender Anlagen.

## Patentansprüche

1. Bioreaktor, insbesondere Fermentationsreaktor, mit einem zylindrischen Reaktortank (1) mit vertikaler Achse (1') und einem in einem Strömungsleitrohr (2) angeordneten, im Wesentlichen vertikal nach unten fördernden Rührwerk (3), sowie einem im Inneren des Reaktortanks (1) angeordneten Wärmetauscher (4), wobei der Wärmetauscher (4) im Bereich der Austrittsöffnung (11) des Strömungsleitrohrs (2) angeordnet ist und in Bezug auf die vertikale Achse (1') des Reaktortanks (1) radial nach außen weisende, vertikale Strömungsleitelemente (10) aufweist, **dadurch gekennzeichnet, dass** mehrere, vorzugsweise alle Strömungsleitelemente (10) jeweils ein von einer ersten Ringleitung (6) gespeistes, oberes Rohr (8) und ein von einer zweiten Ringleitung (7) gespeistes, unteres Rohr (9) aufweisen, wobei die beiden Rohre (6, 8) jedes Strömungsleitelementes (10) zur Zu- und Abfuhr eines Heiz- oder Kühlmediums dienen und zwischen den beiden Rohren (6, 8) ein vertikales Leitblech (12) angeordnet ist.

2. Bioreaktor nach Anspruch 1, **dadurch gekennzeichnet, dass** die Zu- und Abfuhr des Heiz- oder Kühlmediums über das obere (8') und das untere Rohr (9') eines der Strömungsleitelemente (10) erfolgt, dessen beiden Rohre (8', 9') durch die Wand des Reaktortanks (1) hindurchgeführt sind.

3. Bioreaktor nach Anspruch 1 oder 2, **dadurch gekennzeichnet, dass** zwischen der Austrittsöffnung (11) des Strömungsleitrohrs (2) und dem zentralen Bereich des Wärmetauschers (4) ein Strömungsleitkegel (5) zur Umlenkung der vom Rührwerk (3) erzeugten Vertikalströmung (13) in eine nach außen gerichtete Radialströmung (14) angeordnet ist.

4. Bioreaktor nach Anspruch 3, **dadurch gekennzeichnet, dass** für die Zufuhr von Rohsubstraten in den Reaktortank (1) eine im Bereich der Spitze des Strömungsleitkegels (5) einmündende Substratzufuhrleitung (15) vorgesehen ist.

5. Bioreaktor nach einem der Ansprüche 1 bis 4, **dadurch gekennzeichnet, dass** die vertikale Lage der zentralen Eintrittsöffnung (16) des Strömungsleitrohres (2) auf die Höhe des Füllstandes des Substrates im Reaktortank (1) derart abgestimmt ist, dass sich beim Betrieb des Rührwerks (3) an der Substratoberfläche (17) eine Strömungstrombe (18) ausbildet.

6. Bioreaktor nach einem der Ansprüche 1 bis 5, **dadurch gekennzeichnet, dass** das Rührwerk (3) als Tauchrührwerk ausgeführt ist, welches von einem Speichenkranz (19) im Strömungsrohr (2) zentriert ist und sich vorzugsweise an Standfüßen (20) des Strömungsleitrohres (2) abstützt.

7. Bioreaktor nach einem der Ansprüche 1 bis 6, **dadurch gekennzeichnet, dass** eine Baugruppe bestehend aus dem Strömungsleitrohr (2) mit integriertem Rührwerk (3), dem Strömungsleitkegel (5) und dem Wärmetauscher (4) am Boden des Reaktortanks (1) oder bevorzugt direkt am Fundament (21) des Reaktortanks befestigt ist.

8. Baugruppe zum Einbau in einen Bioreaktor, insbesondere Fermentationsreaktor, mit einem zylindrischen Reaktortank (1), wobei die Baugruppe ein Strömungsleitrohr (2) mit integriertem, vertikal nach unten förderndem Rührwerk (3) aufweist, **dadurch gekennzeichnet, dass** die Baugruppe zusätzlich folgende Bestandteile aufweist:
• einen ausgangsseitig des Strömungsleitrohrs (2) angeordneten Strömungsleitkegel (5) zur Umlenkung der vom Rührwerk (3) erzeugten Vertikalströmung (13) in eine nach außen gerichtete Radialströmung (14) und
• einen Wärmetauscher (4) mit radial nach außen weisenden, vertikalen Strömungsleitelementen (10).

9. Baugruppe nach Anspruch 8, **dadurch gekennzeichnet, dass** die Baugruppe am Boden des Reaktortanks (1) oder bevorzugt direkt am Fundament (21) des Reaktortanks befestigbar ist.

## Claims

1. A bioreactor, in particular a fermentation reactor, comprising a cylindrical reactor tank (1) with a vertical axis (1') and a stirring mechanism (3) which is arranged in a flow guide tube (2) and conveys essentially vertically in the downward direction, as well as a heat exchanger (4) arranged in the interior of the reactor tank (1), wherein the heat exchanger (4) is arranged in the region of the outlet opening (11) of the flow guide tube (2) and comprises vertical flow guide elements (10') which face radially to the outside with respect to the vertical axis (1') of the reactor tank (1), **characterised in that** several, preferably all, flow guide elements (10) each have an upper tube (8) fed by a first ring line (6) and a lower tube (9) fed by a second ring line (7), wherein the two tubes (6, 8) of each flow guide element (10) are designated for the supply and removal of a heating or cooling medium, and a vertical guide plate (12) is arranged between the two pipes (6, 8).

2. A bioreactor according to claim 1, **characterised in that** the supply and removal of the heating or cooling medium takes place via the upper (8') and lower tube (9') of one of the flow guide elements (10), the two pipes (8', 9') of which are guided through the wall of the reactor tank (1).

3. A bioreactor according to claim 1 or 2, **characterised in that** a flow guide cone (5) for deflecting the vertical flow (13) produced by the stirring mechanism (3) into an outwardly directed radial flow (14) is arranged between the outlet opening (11) of the flow guide tube (2) and the central region of the heat exchanger (4).

4. A bioreactor according to claim 3, **characterised in that** a substrate supply line (15) opening into the region of the tip of the flow guide cone (5) is provided for the supply of raw substrates to the reactor tank (1).

5. A bioreactor according to one of claims 1 to 4, **characterised in that** the vertical position of the central inlet opening (16) of the flow guide tube (2) is adjusted to the height of the filling level of the substrate in the reactor tank (1) in such a way that a flow vortex (18) is formed on the substrate surface (17) during operation of the stirring mechanism (3)

6. A bioreactor according to one of claims 1 to 5, **characterised in that** the stirring mechanism (3) is designed as an immersion stirring mechanism, which is centred by a spoke rim (19) in the flow tube (2) and preferably rests on pedestals (20) of the flow guide tube (2) supported.

7. A bioreactor according to one of claims 1 to 6, **characterised in that** an assembly consisting of the flow guide tube (2) with integrated stirring mechanism (3), the flow guide cone (5) and the heat exchanger (4) are fastened to the bottom of the reactor tank (1) or preferably directly to the foundation (21) of the reactor tank.

8. An assembly for installation in a bioreactor, especially a fermentation reactor, comprising a cylindrical reactor tank (1), wherein the assembly comprises a flow guide tube (2) with an integrated stirring mechanism (3) conveying vertically downwards, **characterised in that** the assembly additionally comprises the following components:
• a flow guide cone (5) arranged on the outlet side of the flow guide tube (2) for deflecting the vertical flow (13) generated by the stirring mechanism (3) into an outwardly directed radial flow (14), and
• a heat exchanger (4) with vertical flow guide elements (10) that are irected radially to the outside.

9. An assembly according to claim 8, **characterised in that** the assembly can be fastened to the bottom of the reactor tank (1) or preferably directly to the foundation (21) of the reactor tank.

## Revendications

1. Bioréacteur, notamment fermentateur comportant une cuve cylindrique (1) d'axe vertical (1') et un agitateur (3) débitant vers le bas, installé pratiquement verticalement dans un conduit de guidage de courant (2) ainsi qu'un échangeur de chaleur (4) installé à l'intérieur de la cuve (1),
- l'échangeur de chaleur (4) étant situé dans la région de l'orifice de sortie (11) du tube de guidage de courant (2) en ayant des éléments de guidage de courant (10), verticaux, orientés radialement vers l'extérieur par rapport à l'axe vertical (1') de la cuve (1),
**caractérisé en ce que**
plusieurs et de préférence tous les éléments de guidage de courant (10) ont respectivement un tube supérieur (8) alimenté par une première conduite annulaire (6) et un tube inférieur (9) alimenté par une seconde conduite annulaire (7), les deux tubes (6, 8) de chaque élément de guidage de courant (10) servant à l'alimentation et à l'évacuation d'un milieu de chauffage ou de refroidissement et entre les deux tuyaux (6, 8), il y a une tôle de guidage (12) verticale.

2. Bioréacteur selon la revendication 1,
**caractérisé en ce que**
l'alimentation et l'évacuation du milieu de chauffage ou de refroidissement se font par le tuyau supérieur (8') et le tuyau inférieur (9') d'un des éléments de guidage de courant (10) dont les deux tuyaux (8', 9') traversent la paroi de la cuve (1) du réacteur.

3. Bioréacteur selon la revendication 1 ou 2,
**caractérisé en ce qu'**
entre l'orifice de sortie (11) du tube de guidage de courant (2) et la zone centrale de l'échangeur (4), il y a un cône de guidage de courant (5) pour dévier le courant vertical (13) généré par l'agitateur (3) en un courant radial (14) orienté vers l'extérieur.

4. Bioréacteur selon la revendication 3,
**caractérisé en ce qu'**
une conduite d'alimentation en substrat (15) débouchant dans la région de la pointe du cône de guidage de courant (5) assure l'alimentation en substrat brut de la cuve (1) du réacteur.

5. Bioréacteur selon l'une des revendications 1 à 4,
**caractérisé en ce que**
la position verticale de l'orifice d'entrée central (16) du tube de guidage de courant (2) est réglée en fonction de la hauteur du niveau de remplissage du substrat dans la cuve (1), pour que la surface supérieure (17) du substrat forme une trombe (18) lorsque l'agitateur (3) fonctionne.

6. Bioréacteur selon l'une des revendications 1 à 5,
**caractérisé en ce que**
l'agitateur (3) est un mécanisme plongeur centré par une couronne (19) de rayons dans le tube de guidage de courant (2) et il s'appuie de préférence sur les pieds (20) du tube de guidage de courant (2).

7. Bioréacteur selon l'une des revendications 1 à 6,
**caractérisé en ce qu'**
un module composé du tube de guidage de courant (2) intégrant un agitateur (3), le cône de guidage de courant (5) et l'échangeur de chaleur (4), est fixé au fond de la cuve (1) du réacteur ou de préférence directement aux fondations (21) de la cuve du réacteur.

8. Module destiné à être installé dans un bioréacteur, notamment un fermentateur comportant une cuve cylindrique (1), le module ayant un tube de guidage de courant (2) intégrant un agitateur (3), vertical, module **caractérisé en ce qu'**il comprend :
* un cône de guidage de courant (5) installé du côté de la sortie du tube de guidage de courant (2) pour dévier le courant vertical (13) généré par l'agitateur (3) en un courant radial (14) orienté vers l'extérieur, et
* un échangeur de chaleur (4) avec des éléments de guidage de courant (10), verticaux, orientés vers l'extérieur.

9. Module selon la revendication 8,
**caractérisé en ce qu'**
il peut être fixé au fond de la cuve (1) du réacteur ou de préférence directement aux fondations (21) de la cuve de réacteur.
